# EUROPEAN PATENT APPLICATION

(11) **EP 1 346 732 A2**
(43) Date of publication of application: **24.09.2003**
(21) Application number: 03251357.4
(22) Date of filing: 06.03.2003
(51) Int. Cl.: A61K 47/48

(54) **Dosage forms for hygroscopic active ingredients**

(30) Priority: 18.03.2002 US 365273 P
(71) Applicant: ROHM AND HAAS COMPANY, Philadelphia, Pennsylvania 19106-2399 (US)
(72) Inventor: Khanna, Satish Chandra, 4103 Bottmingen (CH); Hughes, Lyn, Harleysville, Pennsylvania 19438 (US)
(74) Representative: Kent, Venetia Katherine

(57) **Abstract**

Dosage forms are disclosed that reduce or eliminate manufacturing and storage stability problems associated with hygroscopicity and deliquescence of active ingredients.

## Description

### BACKGROUND OF THE INVENTION

Water absorption during the manufacture of dosage forms or during storage of dosage forms is problematic. When water absorption occurs during manufacturing the consequences include processing problems such as stickiness, clumping, poor release from dies, poor flow characteristics, chemical instability, and variable product assay. When water absorption occurs during storage the consequences include chemical instability of the active ingredient, change in dissolution characteristics, change in crystalline form, and deterioration of appearance. See "Remington: The Science and Practice of Pharmacy", 20^{th} Edition, pages 708 - 712.

The severity of water absorption or hygroscopicity varies depending on the specific chemical active ingredient. In the most severe cases the chemical active ingredient absorbs water to the point that it liquefies. This is known as deliquescence.

The art is replete with efforts to solve the problem of hygroscopicity and deliquescence.

US 3,903,137 discloses the use of the sulfonate salts of choline as being less hygroscopic than the halide salts of choline. US 5,043,168 discloses the addition of various magnesium and calcium compounds to reduce the deliquescence of choline salicylate. Other methods for stabilizing choline salicylate are disclosed in US 3,801,613, US 3,898,332, US 4,067,974, US 4,147,776, and US 4,338,311,

US 4,626,532 discloses the use of additives, such as cetyl alcohol and cetostearoyl alcohol, to reduce water absorption of bacampicillin.

US 5,486,363 and WO 91/15198 disclose the use of encapsulation to provide a moisture barrier to reduce water absorption. Doekler et al (Congr. Int. Technol. Pharm., 3rd (1983), Volume 5, pages 73-82) describe the use of waxy matrices to protect against water absorption.

EP 0276116 discloses a method for loading solutions of deliquescent and hygroscopic active ingredients into capsules, thus avoiding the need to retain said active ingredient in the solid form.

WO 00/34293 discloses the use of the pentahydrate of sodium pamidronate that is not deliquescent, as an improvement over the amorphous form or other crystalline forms that are deliquescent.

US 6,204,255, WO 96/23491, US 5,212,326, and WO 01/39747 disclose various methods of preparing forms of sodium valproate that are not deliquescent, including the preparation of a sodium valproate-valproic acid complex, and the preparation of sodium valproate-cyclodextrin complexes.

WO 98/54166 discloses the use of tartrate salts of a drug for the treatment of CNS disorders that has reduced hygroscopicity compared to the hydrochloride.

US 3,337,402 and US 4,761,274 disclose the use of adsorbents such as magnesium aluminum silicates to reduce hygroscopicity and deliquescence.

With the exception of the encapsulation methods disclosed in US 5,486,363 and WO 91/15198 none of these techniques can be considered general in their applicability. However, encapsulation can involve significant manufacturing and handling difficulties. For example a specific salt of one active ingredient may be non-hygroscopic, but that same salt of another active ingredient may be deliquescent. Also, the addition of a particular additive used in the encapsulation process may not have the same beneficial effect with different active ingredients.

Thus, the pharmaceutical and industrial formulator must resort to exceptional methods to avoid exposure to normal atmospheric conditions of humidity, such as placing all the formulation equipment in a specially constructed dry-room, which is clearly an inconvenient and costly solution, or scheduling production for periods of low ambient humidity, an approach which seriously curtails productivity.

Furthermore, the uptake of water and /or humidity by active ingredients can lead to severe stability problems during storage.

There is a need for a general technique to solve the aforementioned problems associated with the manufacture and use of dosage forms containing highly hygroscopic or deliquescent active ingredients.

Applicants have surprisingly discovered that dosage forms comprising resinates of ionizable hygroscopic or deliquescent active ingredients solves the problems associated with hygroscopicity and deliquescence. This is particularly surprising because the ion exchange resins used to prepare the resinates are hygroscopic. It is also surprising that increasing the amount of hygroscopic or deliquescent active ingredient in the resinate decreased the water absorption characteristics of the resinate.

The following terms have the following meanings herein:

The term "hygroscopic" or "hygroscopicity", as used herein, describes the property of an active ingredient of absorbing or adsorbing water from the air or surrounding atmosphere. When a "hygroscopic" active ingredient is capable of absorbing or adsorbing water from the air or surrounding atmosphere to the extent that said active ingredient becomes liquid said active ingredient is considered "deliquescent". By this definition as used herein all deliquescent active ingredients are hygroscopic. Deliquescence represents the most severe case of hygroscopicity.

The term "release medium" and as used herein, means the aqueous liquid medium into which the active ingredients is being released. Examples of physiological release media can be simulated intestinal fluid, simulated gastric fluid, simulated saliva, or the authentic physiological versions of these fluids, and /or other release media such as water, and various buffer solutions.

The term "ion exchange resin", as used herein, means any insoluble polymer that can act as an ion exchanger.

The term "release", as used herein, means the transfer of active ingredient from the resinate into the release medium. When applied to a resin or resinate, the term "absorption", as used herein, means the reverse of release, namely the transfer of active ingredient from the medium into the ion exchange resin or resinate.

The term "water retention capacity" as used herein is used to describe the maximum amount of water that an ion exchange resin can retain within the polymer phase and in any pores. (ASTM D2187: Standard Test Methods for Physical and Chemical Properties of Particulate Ion Exchange Resin. Test Method B: Water Retention Capacity)

The term "resinate," as used herein, means a complex formed between an active ingredient and an ion exchange resin. It is also known as a loaded resin. The term "resinate" can also be expressed as an active ingredient/ion exchange resin complex.

Further, ion exchange resins are characterized by their capacity to exchange ions. This is expressed as the "Ion Exchange Capacity." For cation exchange resins the term used is "Cation Exchange Capacity," and for anion exchange resins the term used is "Anion Exchange Capacity." The ion exchange capacity is measured as the number equivalents of an ion that can be exchanged and can be expressed with reference to the mass of the polymer ( herein abbreviated to "Weight Capacity") or its volume (often abbreviated to "Volume Capacity"). A frequently used unit for weight capacity is "milliequivalents of exchange capacity per gram of dry polymer."
This is commonly abbreviated to "meq/g."

Ion exchange resins are manufactured in different forms. These forms can include spherical and non-spherical particles with size in the range of 0.00001mm to 2mm. The non-spherical particles are frequently manufactured by grinding of the spherical particles. Products made in this way typically have particle size in the range 0.0001mm to 0.2mm. The spherical particles are frequently known in the art as 'Whole Bead.' The non-spherical particles are frequently known in the art as 'Powders.'

### STATEMENT OF THE INVENTION

The present invention relates to a dosage form comprising a resinate of a hygroscopic active ingredient.

The present invention further relates to a method for formulating hygroscopic active ingredients comprising preparing a resinate of said hygroscopic active ingredient.

### DETAILED DESCRIPTION OF THE INVENTION

The present invention relates to a dosage form comprising a resinate of a hygroscopic active ingredient.

The present invention further relates to a method for formulating hygroscopic active ingredients comprising preparing a resinate of said hygroscopic active ingredient.

It is known in the art that ion exchange resins are hygroscopic. However, to those skilled in the art the rate and extent of water absorption by the ion exchange resins is not severe enough under normal operating conditions of formulation equipment to preclude their use or to require exceptional methods to avoid exposure to normal atmospheric conditions of humidity. This is clearly demonstrated by the fact that ion exchange resins have been used in pharmaceutical formulation for at least 40 years without need for such exceptional measures.

The resinates of the present invention are hygroscopic, as shown by the data presented in the Examples below, but the level and extent of this hygroscopicity is similar to or less than that of the original ion exchange resin, and so the use of said resinates does not require exceptional methods to avoid exposure to normal atmospheric conditions of humidity. The resinates are not deliquescent.

Ion exchange resins useful in the practice of the present invention include, but are not limited to, anionic exchange resins and cationic exchange resins. Preferably, said resins are suitable for human and animal ingestion when the application is pharmaceutical.

Preferred anionic exchange resins include, but are not limited to, styrenic strongly basic anion exchange resins with a quaternary amine functionality having a weight capacity of 0.1 to 15 meq/g, and styrenic weakly basic anion exchange resins with a primary, secondary, or tertiary amine functionality having a weight capacity of 0.1 to 8.5 meq/g, and acrylic or methacrylic strongly basic anion exchange resins with a quaternary amine functionality having a weight capacity of 0.1 to 12 meq/g, and acrylic or methacrylic weakly basic anion exchange resins with a primary, secondary, or tertiary amine functionality having a weight capacity of 0.1 to 12 meq/g, and allylic and vinylic weakly basic anion exchange resins with a primary, secondary, or tertiary amine functionality having a weight capacity of 0.1 to 24 meq/g.

More preferred anionic exchange resins include, but are mot limited to, styrenic strongly basic anion exchange resins with a quaternary amine functionality having a weight capacity of 0.1 to 6 meq/g, and styrenic weakly basic anion exchange resins with a tertiary amine functionality having a weight capacity of 0.1 to 8.5 meq/g, acrylic or methacrylic strongly basic anion exchange resins with a quaternary amine functionality having a weight capacity of 0.1 to 8 meq/g, and acrylic or methacrylic weakly basic anion exchange resins with a tertiary amine functionality having a weight capacity of 0.1 to 12 meq/g, and allylic and vinylic weakly basic anion exchange resins with primary, secondary, or tertiary amine functionalities having a weight capacity of 0.1 to 24 meq/g.

Most preferred anionic exchange resins include, but are not limited to, styrenic strongly basic anion exchange resins with a quaternary amine functionality with weight capacity of 0.1 to 6 meq/g and acrylic anion exchange resins with a tertiary amine functionality with weight capacity of 0.1 to 12 meq/g. Styrenic strongly basic anion exchange resins with quaternary amine functionalities with weight capacities of 4.0 to 4.5 meq/g are also known as cholestyramine resins.

Preferred cationic exchange resins include, but are not limited to, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g.

More preferred cationic exchange resins include, but are not limited to, styrenic strongly acidic cation exchange resins with a sulfonic acid functionality having a weight capacity of 0.1 to 8 meq/g; and styrenic weakly acidic cation exchange resins with a phenolic acid functionality having a weight capacity of 0.1 to 8.5 meq/g; and acrylic or methacrylic weakly acidic cation exchange resins with a carboxylic or phenolic acid functionality with a weight capacity of 0.1 to 14 meq/g.

Most preferred cationic exchange resins include, but are not limited to, styrenic strongly acidic cation exchange resins with a sulfonic acid functionality with a weight capacity of 0.1 to 8 meq/g, and acrylic or methacrylic weakly acidic cation exchange resin with a carboxylic acid functionality with weight capacity of 0.1 to 14 meq/g.

Ion exchange resins useful in this invention have a moisture content between 0% and the water retention capacity of said resin.

Ion exchange resins useful in this invention are in powder or whole bead form.

Strongly acidic and weakly acidic cation exchange resins useful in the practice of the present invention are in the acid form or salt form or partial salt form.

Strongly basic anion exchange resins useful in this invention are in the salt form.

Weakly basic anion exchange resins useful in this invention are in the free-base form or salt form or partial salt form.

The particle size of resins and resinates useful in the invention will be defined by the desired release rate profile Typical particle sizes are from 0.00001mm to 2mm. The preferred size is 0.001mm to 1mm. The most preferred size is 0.001mm to 1.0mm

Active ingredients useful in the practice of this invention are ionizable, and hygroscopic. They can be hygroscopic to the point that they are deliquescent.

Active ingredients useful in the practice of the present invention include, but are not limited to, pharmaceutically active ingredients, vitamins, flavors, fragrances, water treatment chemicals such as dispersants, corrosion inhibitors, chelants, biocides, and scale inhibitors, and agricultural chemicals including pesticides, herbicides, fertilizers, and nutrients, that are ionizable, and hygroscopic or deliquescent.

While it is not possible with the current state of knowledge in the art to predict the occurrence and severity of hygroscopicity or deliquescence, methods for determining said properties are know to those skilled in the art. These methods can include the use of moisture balances, visual observation of the physical state, and direct measurement of weight gain at varying temperature and humidity. Examples of highly hygroscopic or deliquescent chemical active ingredients used in pharmaceutical arts to which the present invention can be applied include but are not limited to, salts of valproic acid, salts of choline, sodium pamidronate, rivastigmine, bacampicillin, L-carnitine, *dl*-trans-4-[N-(2-*m*-chlorphenylcyclopropyl) carbamoyloxy]-2-butynyltrimethylammonium chloride, benzyl *d-α*-amino-2-imidazolepropionate dihydrochloride, citroflavinoid salts, and the potassium salt of cytidinephosphocholine, moricizine hydrochloride.

The active ingredient component of the dosage form may be present in any amount which is sufficient to elicit a beneficial effect. Preferably, the loading of active ingredient in the dosage form of the present invention is 1-100% of the ion exchange capacity of the resin, more preferably it is 5-100% of the ion exchange capacity of the resin, most preferably it is 10-100% of the ion exchange capacity of the resin.

The oral dosage form of the present invention is prepared by making a resinate of the active ingredient and an ion exchange resin and formulating said resinate into an oral dosage form.

Said resinate can by formulated into any of the oral dosage forms known in the art including, but not limited to, powders, suspension, tablets, pills, and capsules.

Said resinate can be prepared by any of the methods known in the art. The typical method, known to those skilled in the art, for loading ionizable substances onto an ion exchange resin to form the ionizable substance-ion exchange resin complex (i.e. the resinate) is to dissolve an acidic or basic, ionizable substance in water, and then mix it with a suitable ion exchange resin. See, for example, US2,990,332 and "Remington: The Science and Practice of Pharmacy", 20^{th} Edition, page 913.

In addition to the resinate, excipients are used in the manufacture of the oral dosage forms of the present invention. Excipients useful in the practice if this invention include, but are not limited, to preservatives, viscosity agents, sweetening agents, fillers, lubricants, glidants, disintegrants, binders, and coatings.

Preferred preservatives include, but are not limited to, phenol, alkyl esters of parahydroxybenzoic acid, o-phenylphenol benzoic acid and the salts thereof, boric acid and the salts thereof, sorbic acid and the salts thereof, chlorobutanol, benzyl alcohol, thimerosal, phenylmercuric acetate and nitrate, nitromersol, benzalkonium chloride, cetylpyridinium chloride, methyl paraben, and propyl paraben. Particularly preferred are the salts of benzoic acid, cetylpyridinium chloride, methyl paraben and propyl paraben. The compositions of the present invention generally include from 0-2% preservatives.

Preferred viscosity agents include, but are not limited to, methylcellulose, sodium carboxymethylcellulose, hydroxypropyl-methylcellulose, hydroxypropylcellulose, sodium alginate, carbomer, povidone, acacia, guar gum, xanthan gum and tragacanth. Particularly preferred are methylcellulose, carbomer, xanthan gum, guar gum, povidone, sodium carboxymethylcellulose, and magnesium aluminum silicate. Compositions of the present invention include 0-25% viscosity agents.

Preferred sweetening agents include, but are not limited to, sugar, glucose, fructose, malt syrup , cyclamate, saccharine, sorbitol, aspartame, maltitol, sorbitol and xylitol .

Preferred fillers include, but are not limited to, lactose, mannitol, sorbitol, tribasic calcium phosphate, dibasic calcium phosphate, compressible sugar, starch, calcium sulfate, dextrose and microcrystalline cellulose. The compositions of the present invention contain from 0-75% fillers.

Preferred lubricants include, but are not limited to, magnesium stearate, stearic acid, and talc. The pharmaceutical compositions of the present invention include 0-2% lubricants.

Preferred glidants include, but are not limited to, talc and colloidal silica. The compositions of the present invention include from 0-5% glidants.

Preferred disintegrants include, but are not limited to, starch, sodium starch glycolate, crospovidone, croscarmelose sodium, polacrilin potassium, and microcrystalline cellulose. The pharmaceutical compositions of the present invention include from 0-30% disintegrants.

Preferred binders include, but are not limited to, acacia, tragacanth, hydroxypropylcellulose, pregelatinized starch, gelatin, povidone, hydroxypropylcellulose, hydroxypropyl-methylcellulose, methylcellulose, sugar solutions, such as sucrose and sorbitol, and ethylcellulose. The compositions of the present invention include 0.1-10% binders.

Permeable coatings useful in this invention are well know to one skilled in the art and include Eudragit® RL100, and Eudragit® RS100 (Rohm-Pharma Darmstadt, Germany)

Non-permeable coatings useful in this invention are well known to one skilled in the art and include Aquacoat® CPD (FMC Corporation, Philadelphia, PA, USA), Eudragit® E100, Eudragit® L100, Eudragit® S100 (Rohm-Pharma Darmstadt, Germany), Kollicoat® MA 30 DP (BASF Aktiengesellschaft, Ludwigshafen , Germany).

The following non-limiting examples illustrate the practice of the present invention.

### EXAMPLE 1 - Loading of sodium valproate onto ion exchange resins

The following experiments was carried with two different ion exchange resins. The resins were cholestyramine USP (a strongly basic anion exchange resin in the form of a dry powder) and Amberlite® IRA458 (a strongly basic anion exchange resin in the form of wet whole beads). The quantities used are shown in Table 1.

The ion exchange resin was added to a 200ml screw-capped bottle together with 100ml of water. The sodium valproate was then added. The mixture was shaken at room temperature overnight. The mixtures were then filtered and the solid resinate was dried at 60°C *in vaccuo*.

**Table 1**

| **Resin** | **Weight of resin (g)** | **Weight of sodium valproate (g)** | **Volume of water (ml)** | **Yield of resinate (g)** | **Product** |
|---|---|---|---|---|---|
| Cholestyramine USP | 2.0027 | 1.0008 | 100 | 1.9761 | Resinate A |
| Amberlite® IRA458 | 4.0072 | 1.0078 | 100 | 1.9726 | Resinate B |

### EXAMPLE 2 - Loading of valproic acid onto ion exchange resins

The following experiments was carried with two different ion exchange resins. The resins were Amberlite® IRA67 (a weakly basic anion exchange resin in the form of wet whole beads) and colestipol USP (a weakly basic anion exchange resin in the form of a dry powder). The quantities used are shown in Table 2. The ion exchange resin was added to a 100ml screw-capped bottle. If required, water was then added to hydrate the resin, followed by hexane. The valproic acid was then added. The mixture was shaken at room temperature overnight. The mixtures were then filtered and the solid resinates allowed to air dry for an hour, and then dried at 60°C *in vacuo.*

**Table 2**

| **Resin** | **Weight of resin (g)** | **Weight of valproic acid (g)** | **Volume of water (ml)** | **Volume of Hexane (ml)** | **Yield of resina te (g)** | **Product** |
|---|---|---|---|---|---|---|
| Amberlite® IRA67 | 4.0048 | 1.0118 | 0 | 20 | 2.2078 | Resinate C |
| Colestipol | 2.0022 | 1.0047 | 2 | 20 | 2.6815 | Resinate D |

### EXAMPLE 3 - Storage stability of valproate resinates (severe conditions)

One gram of each dry resin and resinate were weighed out accurately on an analytical balance in an aluminum dish along with a sample of sodium valproate . They were dried for two hours @60°C *in vaccuo* and then placed in an incubator was at 40°C and 75% Relative Humidity. After 17 hours the samples were removed from the incubator and weighed and observations on appearance were noted. % weight increases were calculated. The results are summarized in Table 3

**Table 3**

| **Sample** | **Moisture uptake (%)** | **Observation** |
|---|---|---|
| Cholestyramine USP | 25 | Dry Free Flowing |
| Amberlite® IRA-458 | 34.2 | Sticky |
| Amberlite® IRA-67 | 24.8 | Flowable |
| Colestipol USP | 27.6 | Dry Cake |
| Na Valproate | 40.4 | Liquid |
| Resinate A | 24.6 | Dry, free flowing |
| Resinate B | 35.2 | Sticky |
| Resinate C | 18.8 | Sticky |
| Resinate D | 19.6 | Spongy cake |

These results clearly show that all four resinates are hygroscopic, but are not deliquescent.

### EXAMPLE 4 - Storage stability of valproate resinates (ambient conditions)

The samples from Example 3 were re-dried @60°C *in vaccuo* overnight and then exposed to ambient conditions. During the test the ambient temperature was 25°C and relative humidity was 56%. The results are shown in Table 4

**Table 4**

| **Time** | **Observation** |
|---|---|
| 7:00a m | All samples are dry and free flowing |
| 7:30 | Sodium valproate sample is sticky; all others are free-flowing |
| 7:45 | Sodium valproate sample is sticky; IRA67 and Resinate C are clumpy |
| 8:00 | Sodium valproate sample contains liquid. No change in the other samples. |
| 9:00 | Sodium valproate sample more liquid. No change in the other samples. |
| 10:00 | Sodium valproate sample more liquid. No change in the other samples. |
| 11:00 | No change |
| 12:00 | No change |

These results demonstrate that the flow behavior of the resinates is the same as that of the starting resins, and that in most cases the resinates remain free flowing under typical interior ambient conditions.

### EXAMPLE 5 - Effect of loading on water uptake of resinates.

A series of valproate/colestipol resinates were prepared using the procedure for Resinate D, except that the ratio of resin to colestipol USP was varied to give different loading levels. Each of these samples, together with a sample of colestipol USP were dried at 60°C *in vaccuo* for 2 hours and then exposed to an atmosphere at 40°C and 75% relative humidity for 24 hours. The samples were then weighed to determine the amount of moisture absorbed. The results are shown in Table 5.

**Table 5**

| **Valproate loading (g/g dry)** | **Moisture @ 24 hours (%)** |
|---|---|
| 0.26 | 29.5 |
| 0.18 | 32.7 |
| 0.11 | 35.2 |
| 0 | 37.3 |

As shown in Example 5, the tendency to absorb moisture varies inversely with the amount of deliquescent active ingredient loaded on the resin. This is a very unexpected result. One skilled in the art would expect that having more deliquescent or hygroscopic active ingredient present in the resinate would result in an increase in the amount of water absorbed. This is a particularly advantageous aspect of the invention because this permits the amount of resin used to be minimized, thus maintaining low cost.

This data shows the very unexpected result that increasing the amount of deliquescent active ingredient in the resinate decreases the moisture uptake of the resinate.

### Example 6 - Release of valproate from a resinates

The release of valproate from Resinate A was evaluated and compared to that of sodium valproate. The evaluation was performed using a 50ml Millipore flow-thru filtration cell operated at 37°C. The dissolution medium was prepared as described in the US Pharmacopeia 24 monograph for Valproic Acid (page 1733).

An amount for resinate containing the equivalent of 60 mg of sodium valproate was used for one test and 60mg of sodium valproate for the other. Fractions were collected at 20, 40,and 60 minutes. Sample preparation and analysis were done according to the US Pharmacopeia 24 monograph for Valproic Acid (page 1733). The results are summarized in Table 6

**Table 6 -**

| **% Valproate dissolved or released** | | | | |
|---|---|---|---|---|
| **Sample** | **20 mins** | **40 mins** | **60 mins** | **Total release (mg)** |
| Sodium valproate | 81.9 | 97.8 | 100 | 60.1 |
| Resinate A | 62.5 | 93.6 | 100 | 61.0 |

The data demonstrate that the valproate is rapidly and completely released from the resinate under physiologically relevant conditions and compares very favorably with the dissolution of solid sodium valproate.

## Claims

1. A dosage form comprising a resinate of a hygroscopic active ingredient.

2. A dosage form according to Claim 1, wherein the hygroscopic active ingredient comprises 1 to 100% of the ion exchange capacity of the resin used to prepare the resinate.

3. A method for formulating hygroscopic active ingredients comprising preparing a resinate of said hygroscopic active ingredient.

4. A dosage form according to Claim 1, wherein the hygroscopic active ingredient is selected from the group consisting of the salts of valproic acid, the salts choline, the salts of pamidronic acid, salts of moricizine, and the salts of rivastigmine.
